# EUROPÄISCHE PATENTSCHRIFT

(11) **EP 0 369 249 B1**
(45) Veröffentlichungstag und Bekanntmachung des Hinweises auf die Patenterteilung: **29.12.1993**
(21) Anmeldenummer: 89120324.2
(22) Anmeldetag: 03.11.1989
(51) Int. Cl.: C07D 319/06, C07D 319/08

(54) **Verfahren zur Herstellung von cyclischen Kohlensäureestern**
Process for the preparation of cyclic carbonates
Procédé de préparation de carbonates cycliques

(30) Priorität: 16.11.1988 DE 3838752
(43) Veröffentlichungstag der Anmeldung: 23.05.1990
(73) Patentinhaber: BAYER AG, 51368 Leverkusen (DE)
(72) Erfinder: Schön, Norbert, Dr., D-4150 Krefeld (DE); Buysch, Hans-Josef, Dr., D-4150 Krefeld (DE)

(56) Entgegenhaltungen:
- EP-A- 0 057 360
- DE-A- 3 502 106
- US-A- 3 221 025
- US-A- 3 532 715
- Houben-Weyl, Methoden der Organischen Chemie. 4. Auflage, Band E4, 1983, Seiten 83-85

## Beschreibung

Die vorliegende Erfindung betrifft ein Verfahren zur Herstellung cyclischer Kohlensäureester unter Verwendung katalytischer Mengen einer N-heterocyclischen aromatischen Verbindung.

Es ist bekannt, aliphatische Ringcarbonate aus den zugrundeliegenden Diolen und Phosgen unter Zusatz von äquivalenten Mengen einer Base, bevorzugt von Aminbasen, herzustellen (Houben-Weyl, Methoden der organischen Chemie, 4. Auflage, Band E 4 (1983), S. 83 ff.). Während 1,2-Diole auf diese Weise in guten Ausbeuten in die entsprechenden Fünfringcarbonate überführbar sind, erhält man mit 1,3-Diolen und längerkettigen α,ω-Diolen wesentlich geringere Ausbeuten der entsprechenden Ringcarbonate (J. Org. Chem. 24 (1959), 1873). Die Aufarbeitung ist bei diesem Verfahren umständlich, da die gebildeten Aminsalze meist durch wäßrige Extraktion entfernt werden müssen (vgl. J. Am. Chem. Soc. 73 (1951), 5779; US-A-3.532.715), was besonders bei wasserlöslichen und leicht verseifbaren Carbonaten zu Ausbeuteverlusten führt. Ähnlich wie aliphatische Diole lassen sich auch o,o'-Bisphenole mit Phosgen und äquivalenten Mengen einer Stickstoffbase zu cyclischen Kohlensäureestern umsetzen (US 3.221.025).

Anstelle von Phosgen können auch Kohlensäureester zur Herstellung von cyclischen Kohlensäureestern durch Umesterung eingesetzt werden (Houben-Weyl, Band E 4 (1983), S. 88 ff.). Jedoch beinhaltet diese Verfahrensweise einen zusätzlichen Verfahrensschritt, da die für die Umesterung verwendeten Kohlensäureester vorher, beispielsweise aus Phosgen, hergestellt werden müssen.

EP-A-0 057 360 beschreibt bestimmte cyclische Kohlensäurederivate zur Verwendung als Copolymerisationkomponenten und Verfahren zu deren Herstellung.

Gemäß Beispiel 2 dieser Druckschrift wird Di-(trimethylolpropancarbonat)-carbonat durch Umsetzung von 32 g Trimethylolpropanmonocarbonat mit 10,5 g in 50 g Pyridin und anschließende Aufarbeitung unter Ausschütteln von überschüssigem Pyridin erhalten.

DE-A-3 502 106 beschreibt ein Verfahren zur Herstellung von cyclischen, aliphatischen Orthokohlensäureestern durch Umsetzung eines geeigneten Dichlormethanderivats mit aliphatischen Diolen in Gegenwart stöchiometrischer Mengen einer organischen N-haltigen Base.

Schließlich ist in Houben-Weyl, Methoden der organischen Chemie, 4. Aufl., Band E 4 (1983) auf den Seiten 84 und 85 eine tabellarische Übersicht zur Herstellung von cyclischen Kohlensäure-diestern aus Phosgen und Diolen enthalten, wobei die Umsetzung in der Regel in inerten Lösungsmitteln in Gegenwart von Basen durchgeführt wird. Das letztgenannte Dokument enthält auf Seite 85 auch ein Beispiel, bei dem 2-Oxo(naphtho(1,8-d,e)-1,3-dioxin durch Umsetzung von 1,8-Dihydroxynaphthalin mit Phosgen in Gegenwart von Tetramethylharnstoff als Katalysator hergestellt wurde.

Aufgabe der Erfindung war es daher ein Verfahren zur Verfügung zu stellen, das cyclische Kohlensäureester aus Phosgen und Diolen in hohen Ausbeuten liefert, ohne daß eine Verwendung großer (annähernd äquivalenter) Mengen an Basen erforderlich ist und ohne daß eine aufwendige Aufarbeitung durchgeführt werden muß.

Gegenstand der Erfindung ist ein Verfahren zur Herstellung von cyclischen Kohlensäureestern der Formel 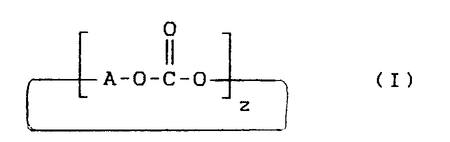
durch Umsetzung von Verbindungen der Formel

HO-A-OH (II),

wobei
- A: der Rest eines aliphatischen Diols mit 3-18 C-Atomen, der Rest eines carbocyclischen oder heterocyclischen nicht-aromatischen Diols mit 3-8 Ringgliedern, der Rest eines Ethergruppen-haltigen aliphatischen Diols mit bis zu 5 Ethergruppen und bis zu 18 C-Atomen oder der Rest einer aromatischen oder araliphatischen Dihydroxyverbindung mit einem oder zwei aromatischen Kernen ist, wobei beide OH-Gruppen in der Formel (II) primäre oder phenolische OH-Gruppen sind und wobei
- z: in Formel (I) für eine ganze Zahl von 1 bis 4 steht
mit Phosgen, in der Schmelze der Dihydroxyverbindung oder in einem inerten Lösungsmittel, dadurch gekennzeichnet, daß man die Umsetzung in Gegenwart von 0,01 bis 5 Gew.-% - bezogen auf Verbindung II - einer stickstoffhaltigen Verbindung K durchführt, bei der wenigstens ein Stickstoffatom Ringglied eines aromatischen Ringes ist.

Der Katalysator K wird vorzugsweise in einer Menge von 0,01-5 Gew.-%, bezogen auf die Verbindung II eingesetzt.

Bevorzugte Verbindungen der Formel (II) sind insbesondere folgende Verbindungen

HO-CH₂-(-CR¹R²-)ₘ-CH₂-OH (III),

gesättigte carbo- bzw. heterocyclische Verbindungen der Formel

HO-CH₂-Q-CH₂-OH (IV),

Ethergruppen-haltige aliphatische Verbindungen der Formel

HO-[(CH₂-)₂-O-]ₒ-(CH₂)₂-OH (V),

und aromatische oder araliphatische Verbindungen der Formeln

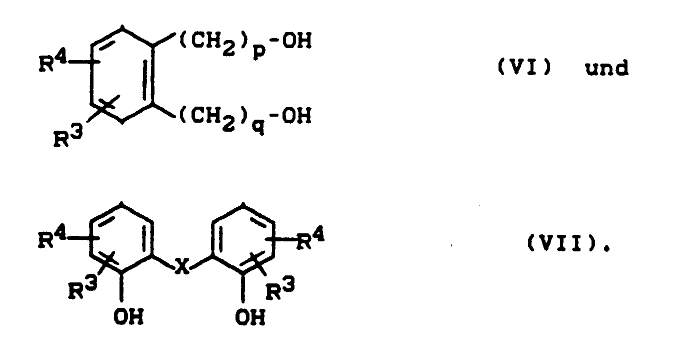

In der Formel (III) hat der Index m die Bedeutung einer ganzen Zahl von 1 bis 16.

R¹ und R² bedeuten unabhängig voneinander Wasserstoff, Alkyl, insbesondere C₁-C₆-Alkyl, Alkenyl, insbesondere Allyl, Aryl, insbesondere Phenyl, Aralkyl, insbesondere Benzyl, wobei die genannten Reste substituiert sein können, oder Halogen, insbesondere Fluor, Chlor, Brom oder Iod, Nitro oder OH. Besonders bevorzugte Reste R¹ und R² sind C₁-C₆-Alkyl, Allyl, Phenyl, Benzyl, Halogen, wie Fluor, Chlor, Brom oder Iod, Nitro, Di(C₁-C₄-alkyl)amino, Hydroxy, Hydroxymethyl, C₁-C₄-Alkoxymethyl, Allyloxymethyl, Halogenomethyl, der Rest der Formel 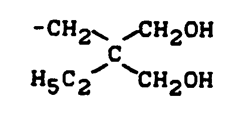
oder gemeinsam Methylen. Die genannten aromatischen Kerne können ihrerseits durch das genannte Halogen, Nitro, Methyl, Ethyl, Methoxy oder Ethoxy substituiert sein.

In der Formel (IV) bedeutet Q ein solches cyclisches System mit 3-8, bevorzugt 3-6 Ringgliedern und kann beispielsweise ein 1,1-verknüpfter Cyclopropan-, Cyclobutan-, Cyclopentan- oder Cyclohexanring, ein 3,3-verknüpfter Oxetan-, Thietan-, Thietan-1-oxid-, Thietan-1,1-dioxidring oder ein 1,2-verknüpfter Cyclobutanring sein.

In der Formel (V) bedeutet der Index o eine ganze Zahl von 1 bis 5, bevorzugt von 1 bis 3.

In der Formel (VI) bedeuten die Indices p und q unabhängig voneinander die Zahlen 0, 1 oder 2, wobei die Summe p + q ≧ 1. Die Reste R³ und R⁴ bedeuten unabhängig voneinander Wasserstoff, C₁-C₆-Alkyl, C₁-C₆-Alkoxy, Halogen, wie Fluor, Chlor oder Brom oder Nitro. R³ und R⁴ können gemeinsam auch einen kondensierten weiteren aromatischen Kern bedeuten. Darüber hinaus kann R³ oder R⁴ auch Phenyl bedeuten.

In der Formel (VII) bedeutet X kann eine Einfachbindung, ein Bindeglied, insbesondere einen gegebenenfalls substituierten Alkylrest, insbesondere Methylen und Ethylen, eine Kohlenwasserstoffkette mit 1-4 C-Atomen, Sauerstoff, Schwefel, -SO- oder -SO₂-. Die Reste R³ und R⁴ haben die genannte Bedeutung.

Verbindungen der Formel (III) können beispielsweise sein 1,3-Propandiol, 1,5-Pentandiol, 1,6-Hexandiol, 1,8-Octandiol, 1.10-Decandiol, 1.12-Dodecandiol, 1.18-Octadecandiol, 2,2-subst. 1,3-Propandiole wie 2-Methyl-1,3-propandiol, 2,2-Dimethyl-1,3-propandiol, 2-Methyl-2-ethyl-1,3-propandiol, 2-Methyl-2-propyl-1,3-propandiol, 2,2-Diethyl-1,3-propandiol, 2-t-Butyl-2-methyl-1,3-propandiol, 2-Butyl-2-methyl-1,3-propandiol, 2-Allyl-2-methyl-1,3-propandiol, 2,2-Diphenyl-1,3-propandiol, 2-Methyl-2-phenyl-1,3-propandiol, 2,2-Dibenzyl-1,3-propandiol, 2-Chlor-2-nitro-1,3-propandiol, Glycerin, 2,2-Bis-hydroxymethyl-1,3-propandiol, 2-Allyloxymethyl-2-ethyl-1,3-propandiol (TMP-monoallylether), 2,2-Bisethoxymethyl-1,3-propandiol (Pentaerythrit-diethylether), 2,2-Bischlormethyl-1,3-propandiol und 2-Methylen-1,3-propandiol.

Beispiele für Verbindungen der Formel (IV) sind Cyclopropan-1,1-dimethanol, Cyclobutan-1,1-dimethanol, Cyclopentan-1,1-dimethanol, 1-Cyclohexen-4,4-dimethanol, Cyclohexan-1,1-dimethanol, Cyclobutan-1,2-dimethanol, 3,3-Bishydroxymethyloxetan und 3,3-Bishydroxymethylthietan.

Beispiele für Verbindungen der Formel (V) und (VI) sind Diethylenglykol, Triethylenglykol, o-Hydroxymethylphenol und 1,2-Bishydroxymethylbenzol.

Geeignete Bisphenole der Formel (VII) sind z.B. gegebenenfalls substituierte 2,2′-Dihydroxybiphenyle, gegebenenfalls substituierte Bis-(2-hydroxyphenyl)-methane wie Bis-(2-hydroxy-5-methylphenyl)-methan, Bis-(3-tert.-butyl-2-hydroxy-5-methylphenyl)-methan, Bis-(5-chlor-2-hydroxyphenyl)-methan, Bis-(3,5-dichlor-2-hydroxyphenyl)-methan, Bis-(3,5-dimethyl-2-hydroxyphenyl)-methan, gegebenenfalls substituierte 2,2-Bis-(2-hydroxyphenyl)-propane wie z.B. 2,2-Bis-(3,5-dimethyl-2-hydroxyphenyl)-propan und gegebenenfalls substituierte Bis-(2-hydroxyphenyl)-oxide und -sulfide.

Besonders geeignete Verbindungen II für das erfindungsgemäße Verfahren sind solche der Formel

HO-B-OH (VIII),

in der
- B: der Rest eines Propan-1,3-diols, der Rest eines Polyethylenglykols oder der Rest eines Bisphenols ist.

Propan-1,3-diole sind hierbei solche der Formel 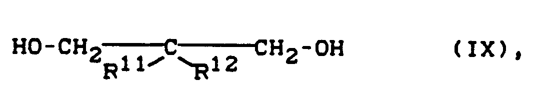
in der
- R¹¹ und R¹²: unabhängig voneinander Wasserstoff, C₁-C₄-Alkyl, Phenyl, Hydroxymethyl,C₁-C₄-Alkoxymethyl oder Allyloxymethyl sein können oder gemeinsam für -CH₂-O-CH₂- oder gemeinsam für C₄- oder C₅-Alkyliden stehen können.

Ebenfalls unter die Formel (VIII) fallende Polyethylenglykole sind solche der Formel

HO-(CH₂-CH₂-O)ᵣ-CH₂-CH₂-OH (X),

in der der Index r die Zahl 1 oder 2 bedeutet.

Die ebenfalls unter die Formel (VIII) fallenden Bisphenole sind solche der Formel (XI) 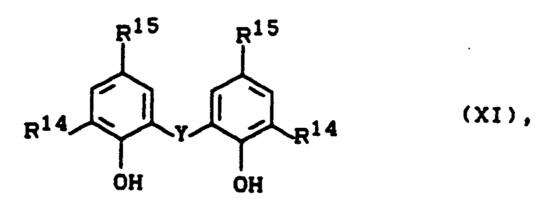
in der
- Y: eine Einfachbindung, Methylen, Sauerstoff oder Schwefel bedeutet und
- R¹⁴ und R¹⁵: unabhängig voneinander Wasserstoff, C₁-C₄-Alkyl oder Chlor bedeuten.

Als typische Vertreter der Dihydroxyverbindungen der Formeln (IX), (X) und (XI) sind ebenfalls wieder beispielhaft und keineswegs erschöpfend zu nennen: 1,3-Propandiol, 2,2-Dimethyl-1,3-propandiol, 2-Ethyl-2-methyl-1,3-propandiol, 2,2-Diethyl-1,3-propandiol, 2-Methyl-2-propyl-1,3-propandiol, 2-Methyl-2-butyl-1,3-propandiol, 2-Methyl-2-phenyl-1,3-propandiol, 2,2-Diphenyl-1,3-propandiol, Trimethylolalkane, wie Trimethylolpropan und Trimethylolethan, Trimethylolpropan-monopropylether, Trimethylolpropan-monoallylether, Cyclopentan-1,1-dimethanol, Cyclohexan-1,1-dimethanol und 3,3-Bis-(hydroxymethyl)-oxetan; Diethylenglykol und Triethylenglykol; 2,2-Dihydroxybiphenyl, Bis-(5-methyl-2-hydroxyphenyl)-methan, Bis-(3,5-dimethyl-2-hydroxyphenyl)-methan, Bis-(5-chlor-2-hydroxyphenyl)-methan und Bis-(2-hydroxyphenyl)-oxid.

Erfindungsgemäß wird in Gegenwart von 0,01-5 Gew.-%, bevorzugt 0,1-2 Gew.-%, bezogen auf die Dihydroxyverbindung, einer Verbindung K gearbeitet. Dies sind vor allem Stickstoffbasen, die das N-Atom in einem aromatischen 5- oder 6-Ring tragen und außerdem keine funktionellen Gruppen tragen, die mit Phosgen, Chlorkohlensäureestern oder Carbonaten unter den Reaktionsbedingungen feste Bindungen eingehen wie z.B. Amino-, Hydroxy- oder Mercaptogruppen. Neben dem N-Atom können im Ringsystem weitere Heteroatome stehen, beispielsweise Sauerstoff, Schwefel oder weitere N-Atome. Der Heterocyclus kann ferner mit anderen aromatischen Heterocyclen oder auch mit aromatischen Carbocyclen kondensiert sein.

Beispiele für solche Verbindungen K sind: Pyridin, Chinolin, Isochinolin; Picoline, Pyrazin, Pyridazin und deren benzokondensierte Abkömmlinge; Triazine wie 2,4,6-Trimethyltriazin, Pyrazol, Imidazol, Triazol und Thiazol sowie die mit C₁-C₄-Alkyl, C₁-C₄-Alkoxy, C₁-C₄-Carbalkoxy oder Halogen substituierte Verbindungen, wie 2-Methyl-imidazol; weiterhin deren benzokondensierte Abkömmlinge, wie Benzimidazol, Benztriazol und Benzthiazol. Die kondensierten Benzolkerne in solchen Systemen können ihrerseits durch C₁-C₄-Alkyl, C₁-C₄-Alkoxy, C₁-C₄-Carbalkoxy, Halogen oder Nitro substituiert sein.

Bevorzugte Verbindungen K für das erfindungsgemäße Verfahren sind Chinoline, Picoline, Benzimidazole, Pyrazole, Triazole und Benzotriazole, Pyridine und Imidazole, speziell Picoline, Pyridin und Imidazol. Selbstverständlich können mehrere dieser Verbindungen als Gemisch eingesetzt werden. Weiterhin können anstelle der freien Basen dieser Verbindungen deren Salze, beispielsweise deren Hydrochloride, Hydrobromide, Sulfate usw. eingesetzt werden. Es können auch solche Salze eingesetzt werden, aus denen sich im Reaktionsgemisch leicht die Hydrochloride bilden können, beispielsweise die Formiate, Acetate, Phosphate, Carbaminate, Picrate und andere.

Die Umsetzung der Dihydroxyverbindung mit Phosgen kann in der Schmelze der Dihydroxyverbindung oder in Lösung erfolgen, wobei inerte Lösungsmittel, wie Kohlenwasserstoffe, Halogenkohlenwasserstoffe, Ester, Nitrile, Ether, Amide usw. eingesetzt werden können. Beispiele für solche Lösungsmittel sind: Dichlormethan, Chloroform, Toluol, Xylol, Chlorbenzol, Dichlorbenzol, Trimethylbenzol, Diphenylether, Chlornaphthalin, Butylacetat, Benzonitril, Dimethylacetamid, N-Methylpyrrolidon,

Die Menge des etwa mitverwendeten Lösungsmittels ist für das erfindungsgemäße Verfahren unkritisch und kann daher in weiten Grenzen schwanken. Beispielsweise können 20 bis 2000 Gew.-%, bezogen auf das Gewicht der eingesetzten Dihydroxyverbindung, bevorzugt 100-1000 Gew.-%, an Lösungsmittel mitverwendet werden.

Zur erfindungsgemäßen Umsetzung werden im allgemeinen 0,8-2 Mol, bevorzugt 0,9-1,5 Mol Phosgen pro Mol Verbindung der Formel II eingesetzt, ganz besonders bevorzugt werden auf 1 Mol Verbindung der Formel II 0,9-1,1 Mol Phosgen eingesetzt, wobei die Komponenten auch nachdosiert werden können. Das Phosgen kann sowohl in einem etwa mitverwendeten Lösungsmittel vorgelegt werden, als auch in Form einer Lösung dem vorgelegten (gelösten oder suspendierten) Dihydroxyverbindung in Form einer Lösung zugegeben werden. Schließlich kann das Phosgen flüssig oder gasförmig ohne weiteres Lösungsmittel in die Lösung, Suspension oder Schmelze der Dihydroxyverbindung einkondensiert werden. Die erfindungsgemäß einzusetzende N-heterocyclische aromatische Verbindung kann sowohl zusammen mit dem Ausgangsmaterial vorgelegt werden als auch nach dem Zusatz des Phosgens zugesetzt werden.

Die erfindungsgemäße Umsetzung wird bei einer Temperatur von -20° C bis +300° C, bevorzugt zwischen 120 und 250° C bei Aromaten und zwischen -20 und 140° C bei Aliphaten durchgeführt. Hierbei kann man so arbeiten, daß die Temperatur im Verlaufe der Umsetzung innerhalb des angegebenen Bereichs gesteigert werden kann. Für den Fall der Mitverwendung eines Lösungsmittels kann dies bei einer solchen Temperatursteigerung abdestilliert werden. Während der erfindungsgemäßen Umsetzung kann man von einem niedriger siedenden Lösungsmittel zu einem höhersiedenden Lösungsmittel übergehen. Dies wird sehr einfach dadurch bewerkstelligt, daß bei einer Temperatursteigerung das niedrigsiedende Lösungsmittel abdestilliert wird, während gleichzeitig kontinuierlich oder absatzweise das höhersiedende Lösungsmittel in den Reaktionsansatz eindosiert wird. Die Umsetzung wird sodann in einem solchen höhersiedenden Lösungsmittel oder bei völligem Abdestillieren aller verwendeten Lösungsmittel auch in der Schmelze des hergestellten cyclischen Kohlensäureesters beendet. Bei der Umsetzung von aliphatischen und Ethergruppen oder cyclischen nicht-aromatische Gruppen enthaltenden Dihydroxyverbindungen hat sich eine Temperatur von -20° C bis +140° C, bevorzugt von -5° C bis +120° C als vorteilhaft erwiesen; hierbei ist es ganz besonders vorteilhaft, wenn die Umsetzung bei einer Temperatur von -5° C bis +50° C, bevorzugt bei -5° C bis +15° C begonnen wird. In der beschriebenen Art läßt sich auch bei dieser Variante die Temperatur während der Umsetzung steigern.

Die erfindungsgemäße Umsetzung ist beendet, wenn auch bei einer Temperatursteigerung eine Chlorwasserstoffentwicklung nicht mehr beobachtet wird. Zur Entfernung von Chlorwasserstoffresten kann es zweckmäßig sein, entweder ein Vakuum anzulegen, oder einen Inertgasstrom (beispielsweise Stickstoff) durch das Reaktionsgemisch zu leiten.

Die Isolierung der cyclischen Kohlensäureester aus dem Umsetzungsgemisch erfolgt in üblicher Weise beispielsweise durch Kristallisation oder Destillation, wobei bei hochsiedenden Reaktionsprodukten auch eine Vakuum- oder Hochvakuum-Destillation in Frage kommt.

Die Ausbeuten an cyclischen Kohlensäureestern sind in der Regel sehr hoch. Im Falle von sechsgliedrigen, aliphatischen Verbindungen sind sie meist größer als 90 % der theoretischen Ausbeute. Die rohen Produkte sind in der Regel für viele Anwendungszwecke bereits rein genug.

Die erfindungsgemäß erhältlichen cyclischen Kohlensäureester sind wertvolle Ausgangsmaterialien für Homo- und Copolycarbonate, Duromersysteme (EP-A 188 204) und Copolymerisate aus cyclischen Kohlensäureestern und Lactamen (DE-A 30 40 612).

### Beispiel 1

### 5,5-Dimethyl-1,3-dioxan-2-on

In eine Suspension aus 208 g (2,00 Mol) 2,2-Dimethylpropan-1,3-diol und 600 ml trockenem Dichlormethan leitete man innerhalb von 1,5 h bei 0 bis 5° C 198 g (2,00 Mol) Phosgen ein und rührte bei dieser Temperatur, bis die anfangs heftige Chlorwasserstoffentwicklung abgeklungen war. Durch einen wirksamen Rückflußkühler (Trockeneis) wurde dabei das Austragen von Phosgen verhindert. Dann wurde das Lösungsmittel im Vakuum entfernt und 500 mg (0,2 Gew.-%) Imidazol und 500 ml Toluol zugesetzt und die Reaktion unter langsamer Steigerung der Temperatur bis auf 110°C innerhalb von 3 h bis zum Abklingen der Chlorwasserstoffentwicklung zu Ende geführt. Chlorwasserstoffreste wurden durch Anlegen eines schwachen Vakuums entfernt. Nach Entfernung des Toluols und Fraktionierung des Rückstands im Vakuum erhielt man 121 g (93 % festes Produkt (Siedepunkt 142 bis 146°C/10 mbar). Nach Umkristallisation aus Ethylacetat erhielt man 117 g (90 %) Produkt (Fp. 108 bis 109° C). Eine weitere Aufarbeitung ist nicht erforderlich.

### Beispiel 2

### Cyclisches Carbonat von 2,2'-Dihydroxybiphenyl

In eine Lösung aus 186 g (1,00 Mol) 2,2'-Dihydroxybiphenyl und 1,00 g Imidazol in 500 ml trockenem Dichlorbenzol wurden innerhalb von 2,5 h bei 115 bis 120° C 150 g (1,50 Mol) Phosgen eingeleitet. Zur Vervollständigung der Chlorwasserstoffabspaltung erhitzte man die Lösung innerhalb von 2 h bis zum beginnenden Rückfluß und destillierte dann das Lösungsmittel im Vakuum ab. Der feste Rückstand (212 g) wurde im Hochvakuum destilliert. Man erhielt 189 g (89 %) eines farblosen kristallisierenden Destillats (Siedepunkt 126 bis 130° C/0,35 bis 0,5 mbar).

Fp. 99 bis 100° C (aus Diisopropylether); IR: 1805 cm⁻¹ (C=O)
Eine weitere Aufarbeitung ist nicht erforderlich.

## Patentansprüche

1. Verfahren zur Herstellung von cyclischen Kohlensäureestern der Formel I 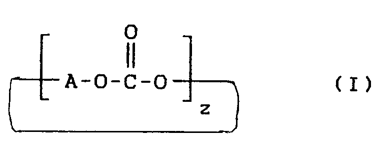 durch Umsetzung von Verbindungen der Formel II
HO-A-OH ,
wobei bedeuten
z eine ganze Zahl von 1 bis 5
A der Rest eines aliphatischen Diols mit 3-18 C-Atomen, der Rest eines nicht-aromatischen Carbocyclus oder Heterocyclus enthaltenden Diols mit 3-8 Ringgliedern, der Rest eines Ethergruppen-haltigen aliphatischen Diols mit bis zu 5 Ethergruppen und bis zu 18 C-Atomen oder der Rest einer aromatischen oder araliphatischen Dihydroxyverbindung mit einem oder zwei aromatischen Kernen ist,
mit Phosgen, in der Schmelze der Dihydroxyverbindung oder in einem inerten Lösungsmittel, dadurch gekennzeichnet, daß die Umsetzung in Gegenwart von 0,01 bis 5 Gew.-% - bezogen auf Verbindung II - einer Verbindung K durchgeführt wird, die wenigstens ein Stickstoffatom als Ringglied in einem aromatischen Ringsystem aufweist.

2. Verfahren nach Anspruch 1, dadurch gekennzeichnet, daß Verbindung II folgender Struktur entspricht
HO-B-OH
in der
B der Rest eines Propan-1,3-diols, der Rest eines Polyethylenglykols oder der Rest eines Bisphenols ist, der nach Entfernung von 2 OH-Gruppen verbleibt.

3. Verfahren nach wenigstens einem der vorhergehenden Ansprüche, dadurch gekennzeichnet, daß die Verbindung K in einer Menge von 0,1-2 Gew.-%, bezogen auf Verbindung II verwendet wird.

4. Verfahren nach wenigstens einem der vorhergehenden Ansprüche, dadurch gekennzeichnet, daß Verbindung K ein Pyridin, Chinolin, Picolin, Imidazol, Benzimidazol, Pyrazol, Triazol oder Benzotriazol ist.

5. Verfahren nach wenigstens einem der vorhergehenden Ansprüche, dadurch gekennzeichnet, daß die Umsetzung in Gegenwart eines inerten Lösungsmittels durchgeführt wird.

6. Verfahren nach wenigstens einem der vorhergehenden Ansprüche, dadurch gekennzeichnet, daß 0,8-2 Mol, Phosgen pro Mol der Verbindung II verwendet werden.

7. Verfahren nach wenigstens einem der vorhergehenden Ansprüche, dadurch gekennzeichnet,daß man die Temperatur im Verlaufe der Umsetzung steigert und bei Verwendung eines Lösungsmittels dieses abdestilliert, wobei man im Verlaufe der Temperatursteigerung das abdestillierte Lösungsmittel durch ein höhersiedendes ersetzt und die Umsetzung im höhersiedenden Lösungsmittel oder bei völligem Abdestillieren aller Lösungsmittel in der Schmelze des Umsetzungsproduktes beendet.

8. Verfahren nach wenigstens einem der vorhergehenden Ansprüche, dadurch gekennzeichnet, daß Verbindung II eine aromatische Verbindung ist und die Umsetzung bei einer Temperatur von 120 bis 250°C durchgeführt wird.

## Claims

1. A process for the production of cyclic carbonic acid esters corresponding to formula I 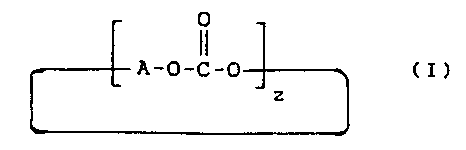 by reaction of compounds corresponding to formula II
HO-A-OH
in which
z is an integer of from 1 to 5 and
A is the residue of an aliphatic diol containing 3 to 18 carbon atoms, the residue of a non-aromatic, carbocyclic or heterocyclic diol containing 3 to 8 ring members, the residue of an ether-functional aliphatic diol containing up to 5 ether groups and up to 18 carbon atoms or the residue of an aromatic or araliphatic dihydroxy compound containing one or two aromatic nuclei,
with phosgene in a melt of the dihydroxy compound or in an inert solvent, characterized in that the reaction is carried out in the presence of 0.01 to 5% by weight - based on compound II - of a compound K which contains at least one nitrogen atom as a member of an aromatic ring system.

2. A process as claimed in claim 1, characterized in that compound II has the following structure
HO-B-OH
in which
B is the residue of a propane-1,3-diol, the residue of a polyethylene glycol or the residue of a bisphenol which remains after the removal of two OH groups.

3. A process as claimed in at least one of the preceding claims, characterized in that compound K is used in a quantity of 0.1 to 2% by weight, based on compound II.

4. A process as claimed in at least one of the preceding claims, characterized in that compound K is a pyridine, quinoline, picoline, imidazole, benzimidazole, pyrazole, triazole or benzotriazole.

5. A process as claimed in at least one of the preceding claims, characterized in that the reaction is carried out in the presence of an inert solvent.

6. A process as claimed in at least one of the preceding claims, characterized in that 0.8 to 2 mol phosgene are used per mol of compound corresponding to formula (II).

7. A process as claimed in at least one of the preceding claims, characterized in that the temperature is increased during the reaction and, where a solvent is used, the solvent is distilled off, the solvent distilled off being replaced by a solvent of higher boiling point during the increase in temperature and the reaction being completed in the solvent of higher boiling point or, where all solvents are completely distilled off, in the melt of the reaction product.

8. A process as claimed in at least one of the preceding claims, characterized in that compound II is an aromatic compound and the reaction is carried out at a temperature in the range from 120 to 250°C.

## Revendications

1. Procédé de production d'esters cycliques d'acide carbonique de formule I 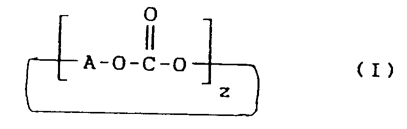 par reaction de composés de formule II
HO-A-OH,
dans laquelle
z est un nombre entier de 1 à 5
A est le reste d'un diol aliphatique ayant 3 à 18 atomes de carbone, le reste d'un diol contenant un carbocycle ou hétérocycle non aromatique, à cycle de 3 à 8 chaînons, le reste d'un diol aliphatique porteur de groupes éther ayant jusqu'à 5 groupes éther et jusqu'à 18 atomes de carbone ou le reste d'un composé dihydroxylique aromatique ou araliphatique ayant un ou deux noyaux aromatiques,
avec le phosgène, dans la masse fondue du composé dihydroxylique ou dans un solvant inerte, caractérisé en ce que la réaction est conduite en présence de 0,01 à 5 % en poids - par rapport au composé II - d'un composé K qui présente au moins un atome d'azote comme chaînon du cycle, dans un système de noyau aromatique.

2. Procédé suivant la revendication 1, caractérisé en ce que le composé II répond à la formule suivante :
HO-B-OH
dans laquelle
B est le reste d'un propane-1,3-diol, le reste d'un polyéthylène-glycol ou le reste d'un bisphénol qui subsiste après élimination de 2 groupes OH.

3. Procédé suivant l'une au moins des revendications précédentes, caractérisé en ce que le composé K est utilisé en une quantité de 0,1 à 2 % en poids par rapport au composé II.

4. Procédé suivant l'une au moins des revendications précédentes, caractérisé en ce que le composé K est la pyridine, la quinoléine, la picoline, l'imidazole, le benzimidazole, le pyrazole, le triazole ou le benzotriazole.

5. Procédé suivant l'une au moins des revendications précédentes, caractérisé en ce que la réaction est conduite en présence d'un solvant inerte.

6. Procédé suivant l'une au moins des revendications précédentes, caractérisé en ce qu'on utilise 0,8 à 2 moles de phosgène par mole de composé II.

7. Procédé suivant l'une au moins des revendications précédentes, caractérisé en ce qu'on fait croître la température au cours de la réaction et, dans le cas de l'utilisation d'un solvant, on chasse ce dernier par distillation, en remplaçant au cours de l'élévation de la température le solvant chassé par distillation par un solvant de plus haut point d'ébullition et en terminant la réaction dans le solvant de plus haut point d'ébullition ou en la terminant dans le produit réactionnel à l'état fondu en chassant entièrement par distillation tous les solvants.

8. Procédé suivant l'une au moins des revendications précédentes, caractérisé en ce que le composé II est un composé aromatique et la réaction est conduite à une température de 120 à 250°C.
